# EUROPEAN PATENT SPECIFICATION

(11) **EP 1 593 347 B1**
(45) Date of publication and mention of the grant of the patent: **05.09.2007**
(21) Application number: 05016337.7
(22) Date of filing: 03.01.2001
(51) Int. Cl.: A61B 17/00

(54) **Apparatus for closing an opening in tissue**
Vorrichtung zum Verschliessen einer Öffnung in einem Gewebe
Dispositif de fermeture d'une overture dans un tissu

(30) Priority: 05.01.2000 US 478179; 11.04.2000 US 546998; 05.07.2000 US 610238
(43) Date of publication of application: 09.11.2005
(62) Divisional of application: 01901754.0
(73) Proprietor: Integrated Vascular Systems, Inc., Abbott Park, IL 60064 (US)
(72) Inventor: Ginn, Richard S., San Jose, CA 95136 (US); Aldrich, William N., Napa, CA 94581 (US); Belef, Martin W., San Jose, CA 95125 (US)
(74) Representative: Viering, Jentschura & Partner

(56) References cited:
- EP-A- 0 774 237
- WO-A-97/07741
- WO-A-99/62408
- US-A- 5 478 354
- US-A- 5 782 861

## Description

### Field Of The Invention

The present invention relates to an apparatus for closing an opening in tissue according to the preamble of independent claim 1. Generally, the present invention relates to an apparatus for closing and/or sealing punctures or other openings in a vessel wall or other body lumen generally formed in conjunction with a diagnostic or therapeutic procedure, and more particularly to introducer sheaths including puncture site closure elements and methods of their use.

### Background Of The Invention

Catheterization and interventional procedures, such as angioplasty and stenting, are generally performed by inserting a hollow needle through a patient's skin and muscle tissue into the vascular system. A guide wire is then passed through the needle lumen into the patient's blood vessel. The needle is removed and an introducer sheath is advanced over the guide wire into the vessel. A catheter is typically passed through the lumen of the introducer sheath and advanced over the guide wire into position for a medical procedure. The introducer sheath therefore facilitates insertion of various devices into the vessel while minimizing trauma to the vessel wall and minimizing blood loss during a procedure.

Upon completion of the medical procedure, the catheter and introducer sheath are removed, leaving a puncture site in the vessel. Commonly, external pressure is applied until clotting and wound sealing occurs. This procedure, however, is time consuming and expensive, requiring as much as an hour of a physician's or nurse's time. It is also uncomfortable for the patient, and requires that the patient be immobilized in an operating room, catheter lab, or holding area. Furthermore, a risk of hematoma exists from bleeding prior to hemostasis.

Various apparatus have been developed for percutaneously sealing a vascular puncture by occluding or suturing the puncture site. For example, an apparatus of the initially mentioned type is known from WO 99/62408, which discloses the preamble of claim 1. Furthermore, U.S. Patent Nos. 5,192,302 and 5,222,974 to Kensey et al., describe the use of a biodegradable plug delivered through the introducer sheath into the puncture site. When deployed, the plug seals the vessel and provides hemostasis. Such devices have been slow to gain acceptance in the medical community, however, due to difficulties encountered in positioning the plug within the vessel. Moreover, the agents used to occlude the puncture site are animal-derived,
typically collagen-based. Thus, a risk of adverse immunoresponse exists with their use. Another previously known technique includes percutaneously suturing the puncture site with specialized apparatus. Such apparatus are described, for example, in U.S. Patent No. 5,304,184 to Hathaway et al. While percutaneous suturing devices may be effective, a significant degree of skill may be required on the part of the practitioner. Also, because such devices are mechanically complex, they tend to be relatively expensive to manufacture.

Surgical staples and resilient clips for external skin wound closure are well known in the art. Examples include U.S. Patent No. 5,026,390 to Brown and U.S. Patent No. 5,683,405 to Yacoubian et al., which both describe resiliently deformable closure devices suitable for manual external application.

To reduce the cost and complexity of percutaneous puncture closure devices, such devices employing resilient clips or staples have been developed. U.S. Patent No. 5,478,354 to Tovey et al. describes the use of resilient clips in conjunction with a trocar to close abdominal puncture wounds. U.S. Patent No. 5,810,846 to Virnich et al. describes a specialized apparatus for closing a vascular puncture site with a plastically deformable clip. The apparatus preferably is advanced over a guide wire through a cannula to the surface of the puncture site, where the staple-like clips are delivered to close the wound.

U.S. Patent No. 5,782,861 to Cragg et al. describes specialized apparatus for closing a puncture site with a detachable clip. The apparatus includes a hollow shaft, having a distal end formed with one or more opposed pairs of resilient grasping prongs, that is advanced over a guide wire through a coaxial hollow tube to a position at the distal end of the tube just proximal of the puncture.

The grasping prongs are extended beyond the distal end of the tube to grasp the vessel on opposing sides of the puncture. The shaft is then partially retracted, causing the prongs to contract within the tube, thereby sealing the puncture site. Both of the devices described in the foregoing patents have the drawback that a separate device must be deployed through the introducer sheath to close the puncture site, thus prolonging the procedure. Moreover, both devices require relatively complex apparatus and involve time consuming manipulation to achieve hemostasis.

The use of backbleed indication as a positioning technique within a vascular puncture is also known. For example, U.S. Patent No. 4,317,445 to Robinson describes a flashback chamber for providing visual indication of venous entry of a cannula. This device, however, does not discuss vascular wound closure. U.S. Patent No. 5,676,689 to Kensey et al., which claims priority from the 5,222,974 patent discussed above, uses a vessel location device to simplify positioning of the biodegradable plug. The vessel locator enables blood from the vessel to flow there through so that the position of the vessel may be determined. The Kensey et al. system, however, only proffers one closure device, and that device is complex and raises concerns about biocompatibility. It also requires the closure component to be positioned within the puncture, thereby increasing the likelihood of dangerous over-advancement of the plug into the vessel.

In view of the foregoing, it would be desirable to provide apparatus suitable for vascular puncture closure that overcome disadvantages of previously known devices.

### Summary of the Invention

The present invention provides an apparatus for closing an opening in tissue having the features of claim 1. Further embodiments are described in the depending claims.

Accordingly, the present invention is directed to an apparatus for closing and/or sealing punctures or other openings in a vessel wall or other body lumen such as those formed during percutaneous or other diagnostic or therapeutic procedures.

When the opposite second ends of the clip pierce a vessel on opposite sides of a puncture site, the introducer sheath is then withdrawn, enabling the clip to contract to its unstressed deployed configuration, thereby drawing opposite sides of the puncture together and closing the wound. Indicators may also be provided for confirming when the spring clip has engaged the vessel wall, thereby indicating to the surgeon that the clip has been deployed and the introducer sheath may be withdrawn.

Advantageously, the wound closure components of the present invention may be inexpensively integrated into a standard-size introducer sheath, thereby eliminating the need for a separate closure device at the conclusion of a catheterization procedure. The present invention provides quick, safe, effective, and easy-to-use apparatus for achieving vascular closure that may overcome drawbacks of previously known devices.

### Brief Description of the Drawings

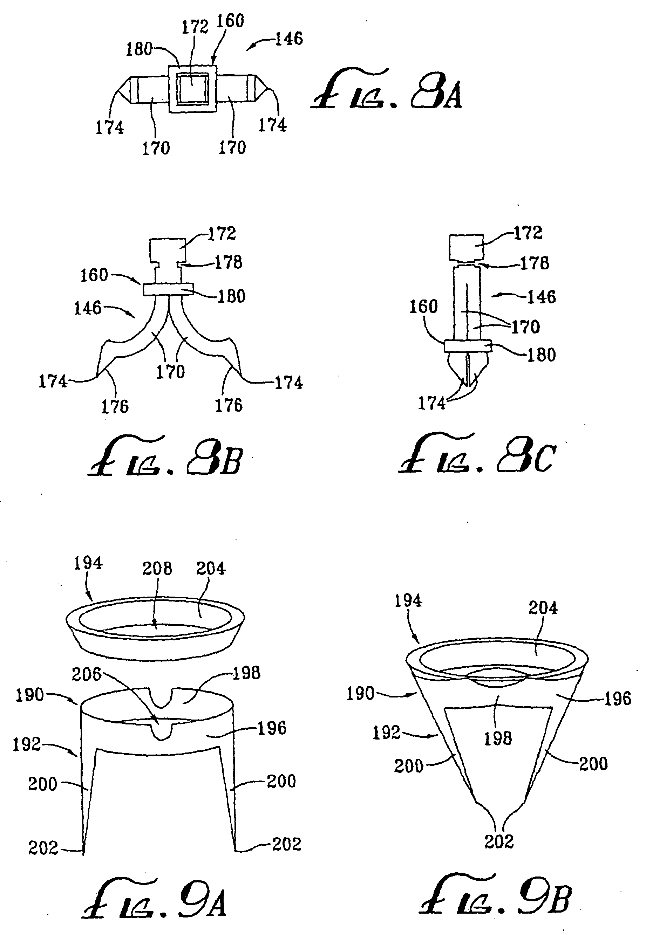

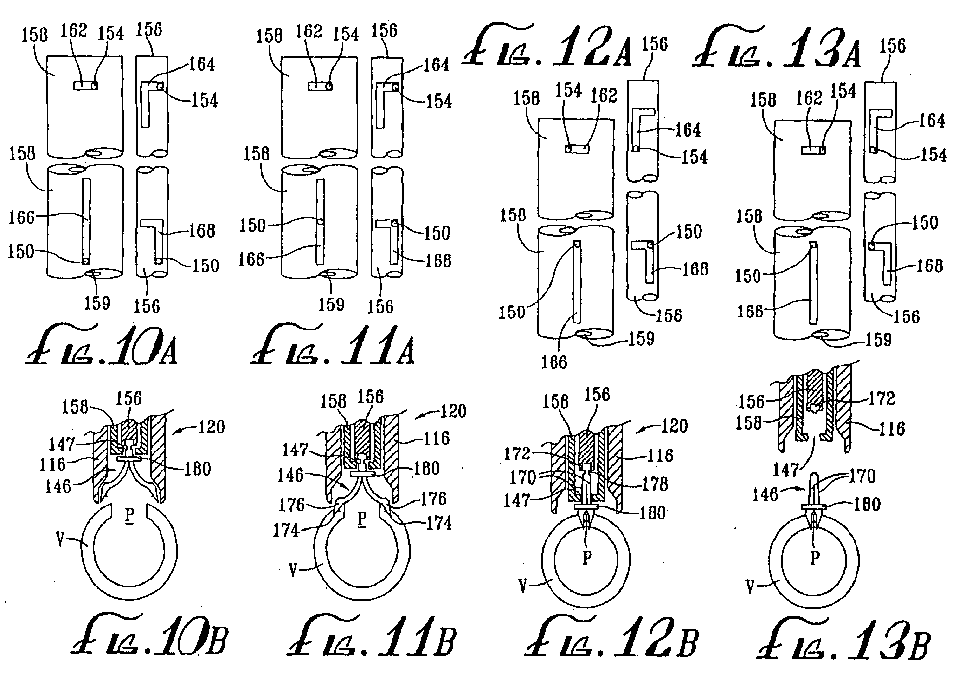

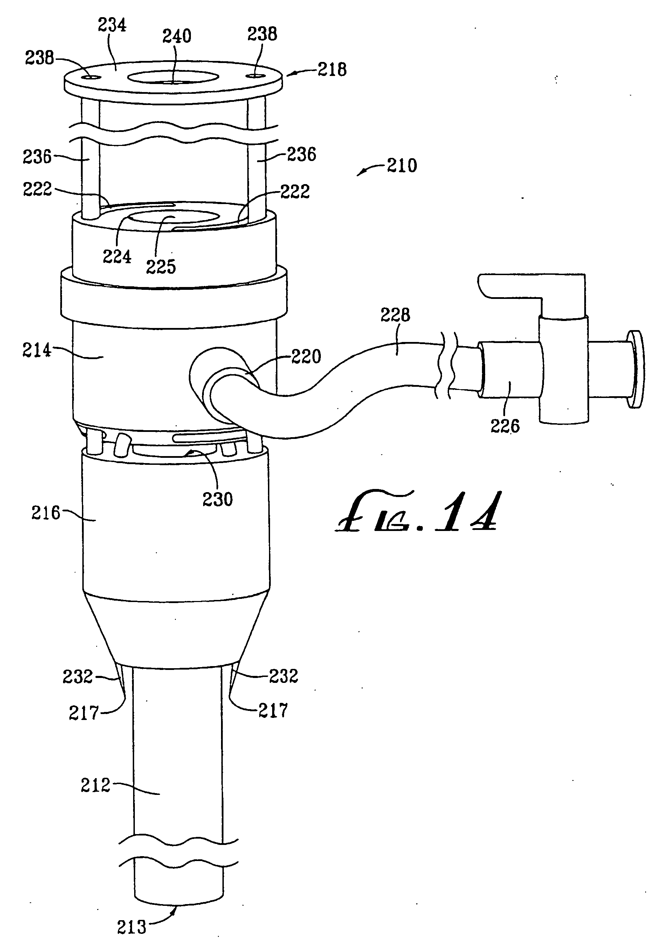

It should be noted that the embodiments of Figs. 14-20b and their respective description are not part of the invention. The above and other objects and advantages of the present invention will be apparent upon consideration of the following detailed description, taken in conjunction with the accompanying drawings, in which like reference characters refer to like parts throughout, and in which:
FIG. 1 is a side view of a vascular device constructed in accordance with the present invention.
FIG. 2 is a cross sectional view of the closure component of the vascular device of FIG. 1.
FIGS. 3A-3D are side views of the resilient clip of the present invention shown from different angles in an expanded delivery configuration and in an unstressed deployed configuration.
FIGS. 4A and 4B are isometric views of an alternative embodiment of the resilient surgical clip, constructed in accordance with the present invention and shown, respectively, in an unstressed deployed configuration and in an expanded delivery configuration.
FIGS. 5A-5F are side-sectional views of a vascular puncture site, illustrating a method not forming part of the invention of sealing the puncture site with the integrated vascular device of FIG. 1.
FIG. 6 is a side view of another integrated vascular device constructed in accordance with the present invention.
FIGS. 7A-7C are, respectively, a cross-sectional view of a closure component of the vascular device of FIG. 6, an exploded side view of proximal slots of the closure component, and an exploded side view of distal slots.
FIGS. 8A-8C are, respectively, views of a bioabsorbable clip and fastener of the present invention shown in top view in a delivery configuration, in side view in the delivery configuration, and in side view in a deployed configuration.
FIGS. 9A and 9B are isometric views of an alternative embodiment of the bioabsorbable surgical clip and fastener, constructed in accordance with the present invention and shown, respectively, in a delivery configuration and in a deployed configuration:
FIGS. 10A-10B through 13A-13B are side-sectional views of the closure component of FIG. 7A in use at a vascular puncture site, with corresponding side views of the proximal and distal slots of FIGS. 7B and 7C, illustrating a method not forming part of the invention for sealing a puncture site.
FIG. 14 is a side view of a preferred embodiment of an integrated vascular device constructed.
FIG. 15 is a side-sectional view of a sealing device for use with the vascular device of FIG. 14.
FIGS. 16A-16D are side views of the closure component of FIG. 14 in use at a vascular puncture site, shown in section, with the sealing device of FIG. 2, illustrating a method not forming part of the invention for sealing a puncture site.
FIGS. 17A-17D are top views of the vascular puncture site of FIGS. 16A-16D, corresponding to the side-sectional views of FIGS. 16A-16D, further illustrating the method of FIGS. 16A-16D.
FIGS. 18A-18C are side-sectional views of an alternative embodiment of an integrated vascular devicein use at a vascular puncture site, illustrating a method not forming part of the invention for sealing a puncture site.
FIGS. 19A-19E are side-sectional views of a further alternative embodiment in use at a vascular puncture site, illustrating a method not forming part of the invention for sealing a puncture site.
FIGS. 20A and 20B are isometric views of a section of vessel including and corresponding to the vascular puncture site of FIGS. 19A-19E, further illustrating the method of FIGS. 19A-19E.

### Detailed Description Of The Invention

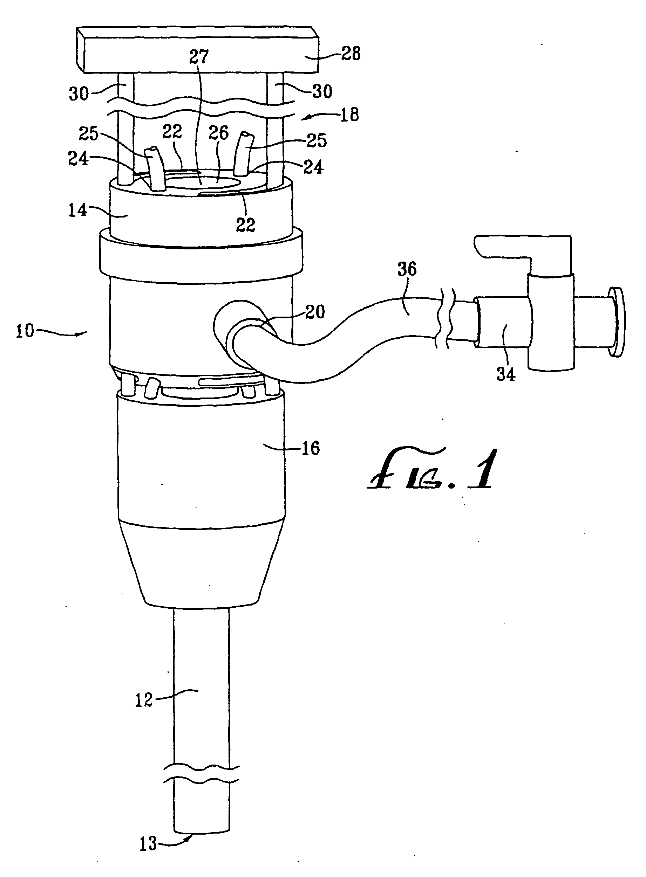

Referring to FIG. 1, a vascular device 10 is provided that includes an introducer sheath 12 coupled to a hub 14, a clip housing 16, and a clip actuator 18. The introducer sheath 12 includes a material typically used for vascular introducer sheaths, such as polyethylene or nylon, and includes a central lumen 13 through which other devices may be introduced in the vasculature, for example, to perform a diagnostic or interventional procedure such as angiography, angioplasty, or stenting.

The hub 14 is mounted to the proximal end of the introducer sheath 12 and includes a side port 20, arc-shaped lumens 22, backbleed lumens 24, backfield tubes 25, and a device port 26. The device port 26 communicates with the central lumen 13 of the introducer sheath 12, and has a self-sealing elastomeric membrane 27 disposed across it. The self-sealing membrane 27, which may include latex or a biocompatible synthetic rubber, permits interventional devices to be introduced through the device port 26, while preventing blood loss through central lumen 13. The side port 20 of the hub 14 is in communication with the central lumen 13, and is connected to a hemostatic port 34 via biocompatible tubing 36.

The clip housing 16 includes an annular-shaped chamber that holds an elastically deformable clip. In accordance with the principles of the present invention, the clip housing 16 is slidably disposed on the exterior of the introducer sheath 12 and is movable from a stowed position, adjacent the hub 14, to a distal clip deployment position, where the clip 62 is urged into engagement with tissue surrounding vascular puncture.

The clip actuator 18 includes a plunger 28 and rods 30, which are configured to slidably pass through the arc-shaped lumens 22 of the hub 14. The distal ends of the rods 30 are mounted in the clip housing 16 such that movement of the plunger 28 causes corresponding proximal or distal movement of the clip housing 16. As described in detail below, when the plunger 28 is moved to its proximal-most position, the clip housing 16 is disposed adjacent to the hub 14 and provides adequate clearance for interventional devices to be inserted through the device port 26 and the central lumen 13 into the patient's vasculature. When moved to its distal-most position, the plunger 28 causes the rods 30 to urge the clip housing 16 distally.

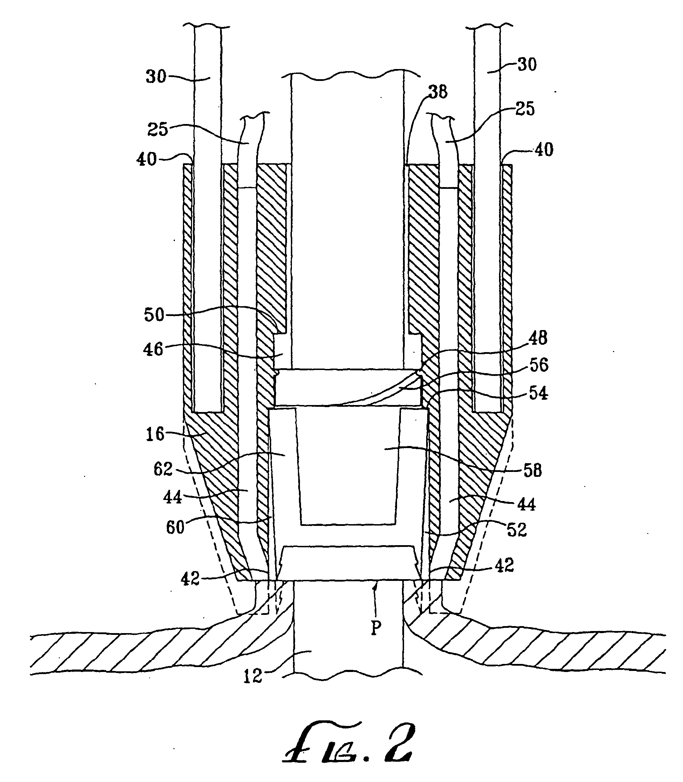

Referring now also to FIG. 2, the vascular device 10 is described in greater detail. The clip housing 16 includes a bore 38 that slidably receives the introducer sheath 12, bores 40 in which the rods 30 are mounted, and backbleed indicator ports 42. The backfield indicator ports 42 are coupled to the backfield tubes 25 via lumens 44. The housing 16 also includes a threaded bore 46 with male threads 48 and a proximal ledge 50, and a clip bore 52 with a proximal ledge 54. The threaded bore 46 engages female threads 56 of a clip expander 58. The clip expander 58 is slidably disposed on the introducer sheath 12, and, together with the portion of clip housing 16 surrounding the clip 62, forms an annular chamber 60.

The clip 62 is stored in its expanded delivery configuration in the annular chamber 60 so that it slidably passes over the clip expander 58 until it abuts the proximal ledge 54 of the clip bore 52. In a delivery configuration of the vascular device 10, the length of the annular chamber 60, as measured from the distal end of the clip expander 58 to the proximal ledge 54, extends within the distal end of the clip housing 16 for a sufficient distance to cover the length of the clip 62. In this manner, the clip housing 16 prevents the clip 62 from snagging on tissue during advancement of the clip housing 16 to its deployed position, as described further below.

The rods 30 pass through the arc-shaped lumens 22 of the hub 14 and are mounted in the bores 40 of the clip housing 16. Distal advancement of the rods 30 causes the clip housing 16, the clip expander 58, and the clip 62 to advance distally a corresponding distance relative to the introducer sheath 12. When the plunger 28 is moved to its distal-most position, the rods 30 may be rotated within the arc-shaped lumens 22 to rotate and advance the clip housing 16 relative to the clip expander 58. This motion causes the clip housing 16 to advance distally along the female threads 56 of the clip expander 58 until the proximal end of the clip expander 58 contacts the proximal ledge 50 of the threaded bore 46. Further rotation of the rods 30 causes the proximal ledge 54 to urge a tissue-engaging portion of the clip 62 distally off of clip expander 58. With the clip housing 16 positioned at a vascular puncture site P, rotation of the rods 30 causes the tissue-engaging portion, e.g., spikes, to pierce the vessel wall, as seen in dotted profile in FIG. 2.

In alternative embodiments, the plunger 28 and rods 30 may be removably coupled to the clip housing 16, e.g., to permit unobstructed access to the device port 26. In this embodiment, the rods 30 may include teeth that may be rotated to fixedly engage the bores 40 in the clip housing 16.

As discussed above, the backbleed indicator ports 42 may be coupled to the tubes 25 via the blood lumens 44 that extend through the clip housing 16. The backfield tubes 25 are slidably disposed through the backfield lumens 24 of the hub 14. When the distal end of the clip housing 16 is advanced distally against the vessel wall at puncture P, blood may enter the blood indicator ports 42 and exit the tubes 25, providing visual confirmation to the surgeon that the distal end of the clip housing 16 is positioned adjacent to the vessel wall. Thus, the backfield tubes 25 may enable the surgeon to determine when the clip housing 16 has been advanced sufficiently to permit clip deployment, while reducing the risk that the clip 62 is either deployed short of the puncture site or extended into the vessel.

Still referring to FIG. 1, in conjunction with clip deployment, a bioglue or tissue sealant may be delivered through the hemostatic port 34, tubing 36, the port 20 and the central lumen 13 of the introducer sheath 12 to vascular puncture P to further help seal the vessel after deployment of the clip 62. Alternatively, the bioglue or tissue sealant may be delivered through the backbleed path described above.

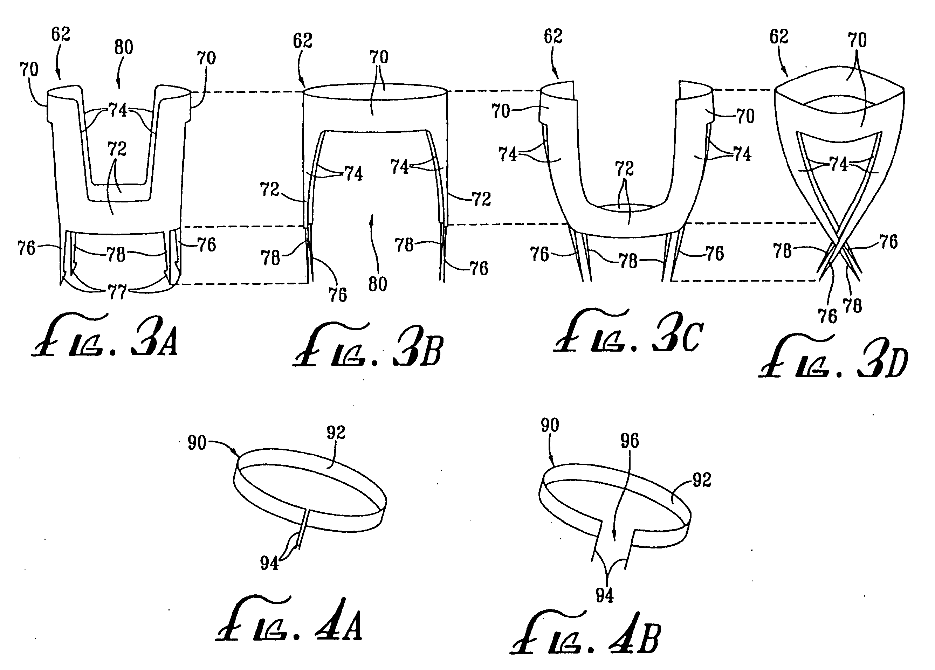

Referring now to FIGS. 3A-3D, an illustrative spring clip 62 constructed in accordance with the principles of the present invention is described in greater detail. FIG. 3B is a side view of the clip of FIG. 3A rotated 90 degrees, wherein the clip 62 is in an expanded delivery configuration. The clip 62 includes an annular device having upper members 70 joined to lower members 72 by legs 74 to form a lumen 80. Outer spikes 76 and inner spikes 78 are connected to the lower members 72, and act as elongated tissue-engaging members. The clip 62 is elastically expanded by advancing the introducer sheath 12 or clip expander 58 (not shown in FIGS. 3A-3D) through the lumen 80.

Upon removal of the introducer sheath, the clip 62 resiliently returns to its unstressed deployed configuration, illustrated in FIGS. 3C and 3D, where FIG. 3C corresponds to the view of FIG. 3A and FIG. 3D corresponds to the view of FIG. 3B. When removed from the exterior of the introducer sheath 12, the clip 62 resumes its deployed shape, in which the opposing sides of the clip come together until lower members 72 contact one another, and outer spikes 76 cross inner spikes 78. As depicted in FIG. 3A, clip 62 also may optionally include engagement elements 77, such as barbs or hooks, to securely engage the vessel being closed.

The clip 62 is preferably fabricated from a superelastic material, such as a nickel-titanium alloy, but may include any material with sufficient resilience to elastically expand for delivery over the introducer sheath 12 and fit within the annular chamber 60 of the clip housing 16 (see FIG. 2). The clip 62 may also be fabricated from a bioabsorbable material or a combination bioabsorbable and elastically expandable material.

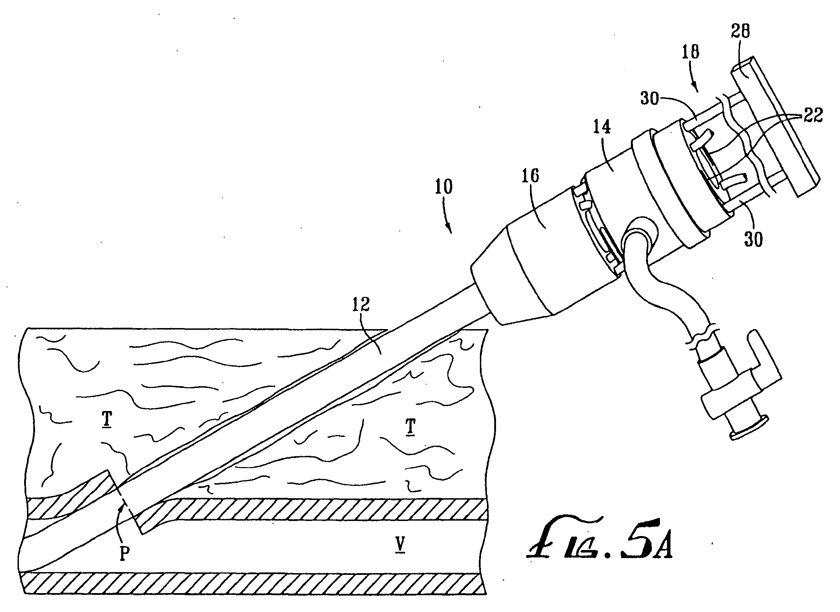

FIGS. 4A and 4B illustrate an alternative embodiment of a resilient spring clip 90 of the present invention, that includes a hoop 92 and opposing spikes 94. In FIG. 4A, the clip 90 is depicted in its unstressed, deployed configuration, in which opposing spikes 94 contact one another. In FIG. 4B, the clip 90 is depicted in its expanded, delivery configuration, in which the opposing spikes 94 are separated by a gap 96. The clip 90 is elastically expanded in a manner similar to the clip 62 described above, e.g., by advancement over an introducer sheath, and preferably also is fabricated from similar materials to those described above.

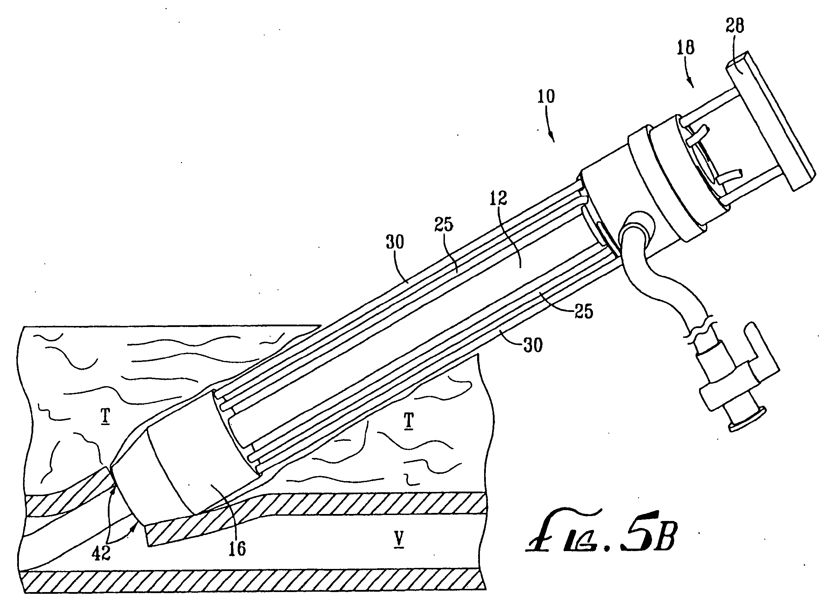

Referring now to FIGS. 5A-5F in conjunction with FIGS. 1-3, methods of using a vascular device 10 are described. In FIG. 5A, the introducer sheath 12 has been advanced through skin, fat, and muscle tissue T into vessel V, through vascular puncture P, which is formed in accordance with well-known techniques. With the plunger 28 and rods 30 in their proximal-most, fully retracted position, an interventional procedure may then be performed by introducing one or more interventional devices, e.g. angioplasty balloons, stent delivery systems, atherectomy devices, etc., through the device port 26 and lumen 13 of the introducer sheath 12 in accordance with well known techniques. The side port 20 may be used to infuse fluids, e.g., contrast agents or medications, into the vessel through the introducer sheath 12 during the interventional procedure.

Upon completion of the procedure, a clip 62 may be used to close vascular puncture P. At this point, the clip actuator 18, the housing 16, the clip expander 58, and the clip 62 are disposed in their proximal-most position adjacent to the hub 14, as depicted in FIG. 5A.

As illustrated in FIG. 5B, the clip actuator 18 may then be advanced by urging the plunger 28 in the distal direction, thereby causing the rods 30 to slide through the arc-shaped lumens 22 of the hub 14 and advance the clip housing 16. Continued distal advancement of the plunger 28 causes the distal end of the clip housing 16 to abut against the exterior of the vessel, so that the backbleed indicator ports 42 of the clip housing 16 directly communicate with the puncture wound. The presence of pressure in the vessel higher than atmospheric pressure causes blood to pass through the indicator ports 42, the blood lumens 44, and exit through the proximal ends of the tubes 25, thereby confirming that the clip housing 16 is positioned at the puncture site and should not be advanced further.

In FIG. 5C, with the clip housing 16 held immobile, the clip actuator 18 is rotated clockwise within the arc-shaped lumens 22 so that the rods 30 rotate and advance the clip housing 16 with respect to the clip expander 58 (see FIG. 2). Specifically, the ledge 54 of the housing 16 contacts the proximal end of the clip 62 and drives the clip 62 distally so that its tissue-engaging members, spikes 76, 78, contact and pierce the wall of vessel V at points around the puncture site, as discussed above with respect to FIG. 2.

Once the spikes 76, 78 have pierced the vessel wall, the clip actuator 18 is rotated counterclockwise within the arc-shaped lumens 22 to retract the clip housing 16, via the threaded bore 46 along the clip expander 58. The tissue-engaging members 76, 78 of the clip 62 retain the clip 62 within the wall of the vessel V while the housing retracts, as shown in FIG. 5D.

In FIG. 5E, with the clip 62 engaged with the vessel wall, the clip housing 16 and the clip expander 58 are withdrawn proximally by proximally withdrawing actuator 18, thereby causing the clip 62 to slide off of the clip expander 58. In FIG. 5E, the spike 78 is embedded in tissue not shown, because that tissue lies within the plane of the cross section.

The vascular device 10 may then be withdrawn from the vessel wall. Once the introducer sheath 12 is removed from the lumen 80 of the clip 62, the clip 62 rotates relative to the vessel wall, as shown in FIG. 5F, and returns to its unstressed, deployed configuration, thus drawing opposite sides of puncture P together to seal the puncture. At this point, a suitable biocompatible bioglue or tissue sealant optionally may be injected into the puncture tract, as discussed above, through the device port 26 or side port, to aid in sealing vascular puncture P. Alternatively, the bioglue or tissue sealant may be delivered through the backbleed path described above.

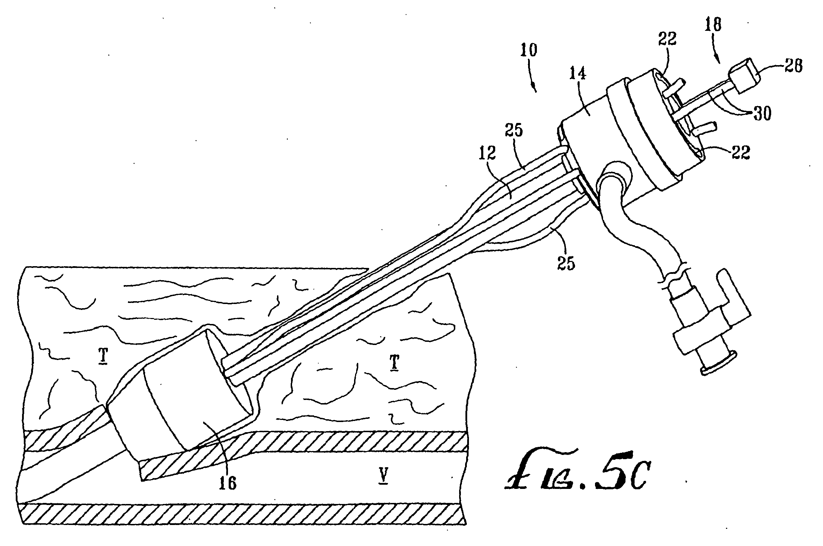

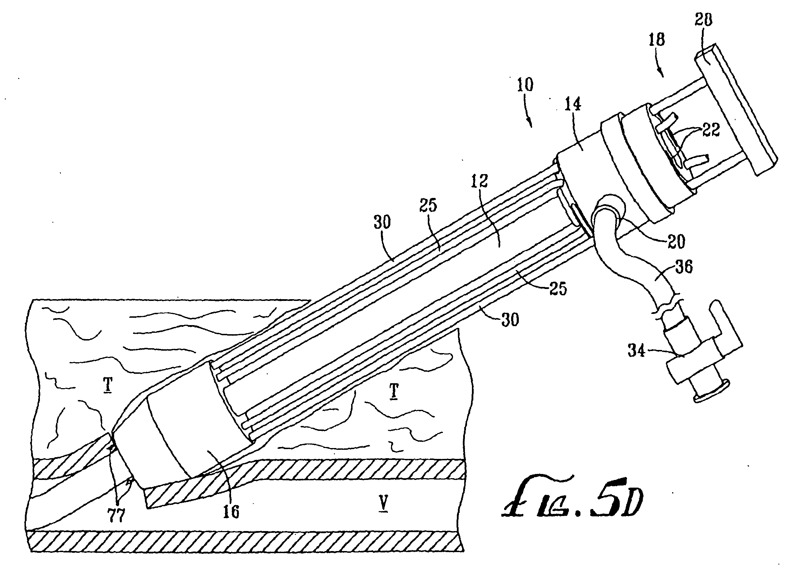

Turning now to FIGS. 6 and 7A, a second embodiment of an apparatus 110 in accordance with the present invention is shown. The apparatus 110 includes an introducer sheath 112 coupled to a hub 114, a clip housing 116, and a clip actuator 118. A closure component 120, described in detail below, is disposed in the clip housing 116. The introducer sheath 112 is formed from a material typically used for vascular introducer sheaths, such as polyethylene or nylon, and includes a central lumen 113 through which other interventional devices may be introduced into the vasculature, for example, to perform a diagnostic or interventional procedure such as angiography, angioplasty, or stenting.

The hub 114 is mounted to the proximal end of the introducer sheath 112 and includes a side port 122, actuator lumens 124, closure lumens 126, backbleed lumens 128, backfield tubes 130, and a device port 132. The device port 132 communicates with the central lumen 113 of introducer sheath 112, and has a self-sealing elastomeric membrane 133 disposed across it. The self-sealing membrane 133, which may be formed from latex or a biocompatible synthetic rubber, may permit interventional devices to be introduced through the device port 132, while preventing blood loss through the central lumen 113. The side port 122 of the hub 114 is also in communication with the central lumen 113, and is connected to a hemostatic port 134 via biocompatible tubing 136.

The clip housing 116 includes two lumens 159 that each hold a bioabsorbable, deformable clip 146. The clip housing 116 is slidably disposed on the exterior of introducer sheath 112 and is movable from a stowed position, adjacent hub 114, to a distal clip deployment position, where the bioabsorbable clips 146 are urged into engagement with tissue surrounding a vascular puncture (not shown). The clip housing 116 prevents the clips 146 from snagging on tissue during advancement of clip housing 116.

The clip actuator 118 includes a plunger 138 and rods 140, which are configured to slidably pass through the actuator lumens 124 of the hub 114. The plunger 138 further includes openings 139. The distal ends of the rods 140 are .ounted in the clip housing 116, so that movement of the plunger 138 causes corresponding proximal or distal movement of the clip housing 116. When the plunger 138 is moved to its proximal-most position, the clip housing 116 is disposed adjacent to the hub 114 and provides adequate clearance for interventional devices to be inserted through the device port 132 and central lumen 113 into the patient's vasculature. When moved to its distal-most position, the plunger 138 causes the rods 140 to urge the clip housing 116 distally.

With particular reference to FIGS. 7A-7C, the clip housing 116 includes a lumen 142 that slidably receives the introducer sheath 112, rod bores 141 (see FIG. 6) in which the rods 140 are mounted, clip lumens 144 in which the clips 146 are housed and advanced to a puncture site, pin holes 148 for rigidly receiving distal pins 150, and backbleed indicator ports (not shown, out of the plane of the cross-section of FIG. 7A) that are coupled to the backbleed tubes 130 via blood lumens 131.

The closure component 120 includes caps 152 with pin holes (not shown, out of the plane of the cross-section of FIG. 7A) configured to receive proximal pins 154, clip holders 156 attached to the clips 146, and locking collar drivers 158 configured to advance fasteners 160. The locking collar drivers 158 are slidably received within lumens 139 of the plunger 138, the closure lumens 126 of the hub 14, and the clip lumens 144 of the clip housing 116. The drivers 158 also include lumens 159 and square clip bores 147, in which clip holders 156 and the clips 146, respectively, are slidably received. The bores 147 preferably have square cross sections.

As illustrated in FIG. 7B, the locking collar drivers 158 include proximal driver slots 162 that communicate with lumens 159, while the clip holders 156 include proximal holder slots 164. The proximal pins 154, mounted in caps 152, pass through and are slidably received within the slots 162 and 164. As seen in FIG. 7C, the locking collar drivers 158 also include distal driver slots 166 that communicate with the lumens 159, while the clip holders 156 further include distal holder slots 168. The distal pins 150, mounted in the clip housing 116, pass through and are slidably received within the slots 166 and 168.

As discussed above, backbleed indicator ports (not shown) are coupled to the backbleed tubes 130 via the blood lumens 131 that extend through the clip housing 116. The backbleed tubes 130 are slidably disposed through the backbleed lumens 128 of the hub 114. When the distal end of the clip housing 116 is advanced distally against a vessel wall at a vascular puncture, blood enters the backbleed indicator ports and exits through the tubes 130, providing visual confirmation to an operator that the distal end of the clip housing 116 is positioned adjacent to the vessel wall. The backbleed tubes 130 thus enable the operator to determine when the clip housing 116 has been sufficiently advanced to permit clip deployment, while reducing the risk that the clips 146 are either deployed short of the puncture site or extended into the vessel.

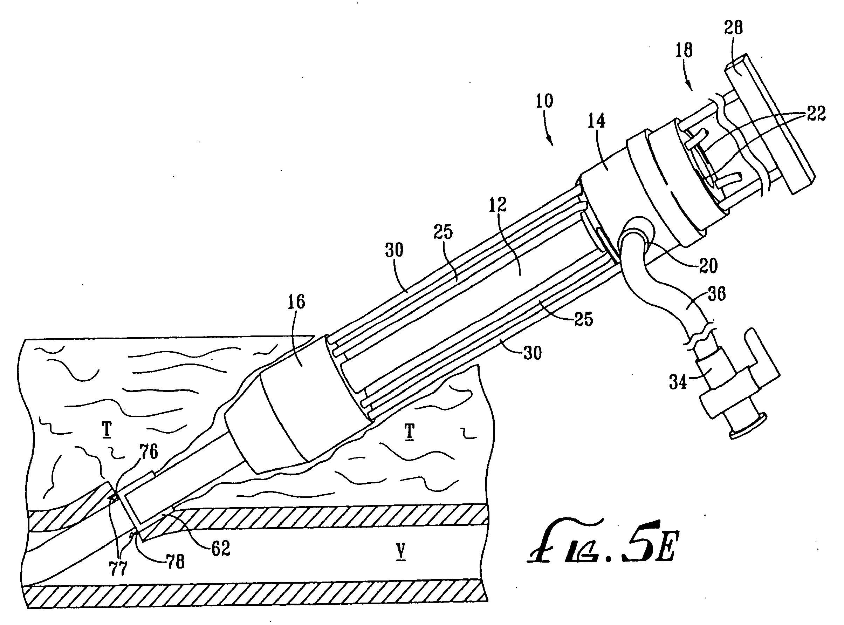

In conjunction with clip deployment, a bioglue or tissue sealant may be delivered through the hemostatic port 134, the biocompatible tubing 136, the side port 122 and the central lumen 113 of the introducer sheath 112 to the vascular puncture to further help seal the vessel after deployment of clips 146. Alternatively, the bioglue or tissue sealant may be delivered through the device port 132 or through the backbleed path described above. With reference now to FIGS. 8A-8C, the clip 146 and fastener 160 are described in greater detail. FIG. 8A shows the clip 146 in its delivery configuration. The clip 46 includes curved legs 170 and proximal end 172. The legs 170 distally terminate at spikes 174 with optional engagement elements 176, such as barbs or hooks, and proximally terminate at narrowed region 178. As seen in FIG. 7A, the proximal end 172 may be attached to the clip holder 156, for example, using an adhesive, and is slidably received by the square clip bore 147 of the locking collar driver 158. As with the bore 147, the clip 146 is of substantially square cross section.

The fastener 160 includes a bioabsorbable locking collar 180, which is slidably received on the exterior of the clip 146. As seen in FIG. 8B, the locking collar 180 may be distally advanced down the exterior of the clip 146 to deform the clip 146 to its deployed configuration, wherein the curved legs 170 and spikes 174 are drawn together. The clip 146 may then be separated from the clip holder 156 by rotating the proximal end 172 with respect to the legs 170, causing the clip 146 to snap into two pieces at the narrowed region 178, as described below. The clip 146 and the locking collar 180 are preferably fabricated from bioabsorbable materials, such as polyglycolic acid.

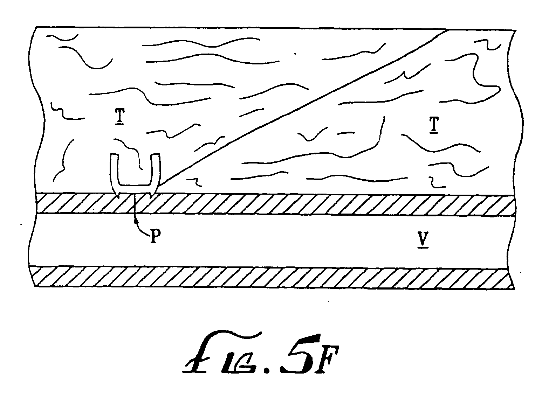

Turning to FIGS. 9A and 9B, an alternative embodiment of a closure component 190 in accordance with the present invention is shown. The closure component 190 includes a bioabsorbable clip 192 and a fastener 194. The clip 192 includes a proximal hoop 196 with narrowed regions 198, and legs 200 terminating in spikes 202. The fastener 194 includes a bioabsorbable wedge 204. The wedge 204 has a diameter substantially equal to the diameter of the hoop 196 at its distal end, the diameter tapering to a maximum diameter at the proximal end of the wedge 204. The clip 192 therefore may be deformed from the delivery configuration of FIG. 9A to the deployed configuration of FIG. 9B, wherein the legs 200 and spikes 202 are drawn together, by advancing wedge 204 into the hoop 196 to deform the clip 192 at the narrowed regions 198. A lumen 206 extends through the hoop 198 of the clip 192, while a lumen 208 extends through the wedge 196. The clip 192 and wedge 196 are thus configured for delivery over the exterior of an introducer sheath (not shown). The clip 192 and wedge 196 are preferably fabricated from bioabsorbable materials.

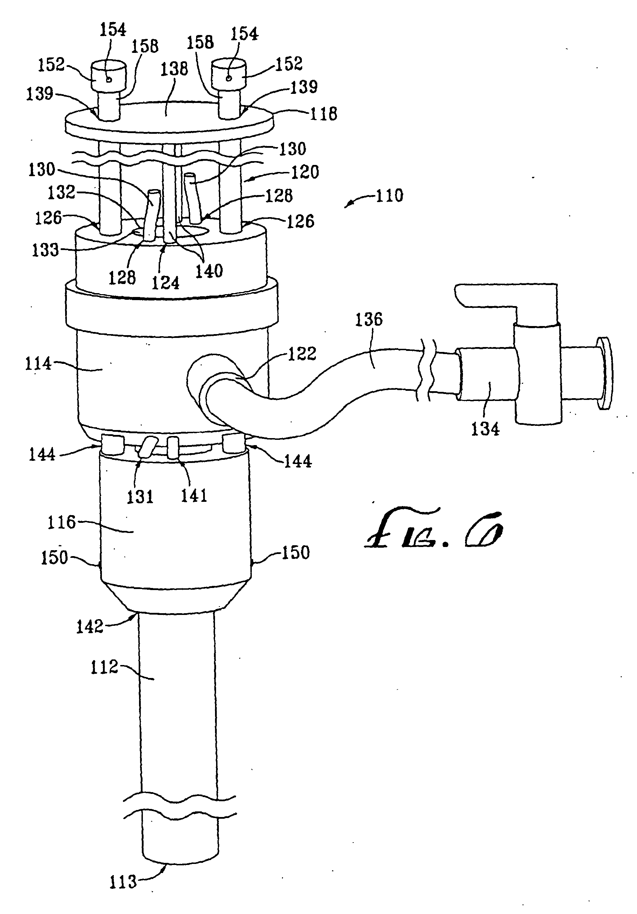

With reference to FIGS. 10A-10B through 13A-13B, in conjunction with FIGS. 6-8C, methods of using the vascular device 110 are described the introducer sheath 112 is advanced through skin, fat, and muscle tissue into vessel V, through vascular puncture P, which is formed in accordance with well known techniques. The vascular device 110 is used in the same manner as a standard introducer sheath, with instruments being advanced into the vessel via lumen 113. Specifically, with the plunger 138 and rods 140 in their proximal-most, fully retracted position, an interventional procedure may be performed by introducing one or more interventional devices, e.g. angioplasty balloons, stent delivery systems, devices, etc., through the device port 132 and the lumen 113 of the introducer sheath 112 in accordance with well-known techniques. The side port 122 may be used to infuse fluids, e.g., contrast agents or medications, into the vessel through the introducer sheath 112 during the interventional procedure.

Upon completion of the procedure, the vascular device 110 may be used to close the vascular puncture P. At this point, the clip actuator 118, the clip housing 116, and the closure component 120 with clips 146, are disposed in their proximal-most position adjacent to the hub 114.

The clip actuator 18 is advanced by urging the plunger 138 in the distal direction, thereby causing the rods 140 to slide through the actuator lumens 124 of the hub 114 and advance the clip housing 116. The distal pins 150, mounted in housing 116, abut the distal slots 166, 168 of the drivers 158 and the holders 156, respectively. Thus, distal advancement of the clip housing 116 also distally advances the closure component 120. Continued distal advancement of the plunger 138 causes the distal end of the clip housing 116 to abut against the exterior of the vessel, so that the backbleed indicator ports (not shown) of the clip housing 116 directly communicate with the puncture wound. The presence of pressure in the vessel higher than atmospheric pressure causes blood to pass through the indicator ports, through the blood lumens 131, and exit through the proximal ends of the tubes 130, thereby confirming that the clip housing 116 is positioned at the puncture site and should not be advanced further.

FIG. 10B illustrates the closure component 120 via sectional views through the clip housing 116 along planes parallel to the introducer sheath 112. FIG. 10A shows the locations of the proximal pins 154 within the proximal slots 162, 164, and the locations of the distal pins 150 within the distal slots 166, 168, corresponding to the relative longitudinal positions of the clip holders 156 and the locking collar drivers 158 depicted in FIG. 10B. The pin locations are shown via side views of the clip holders 156 and the locking collar drivers 158 at the relevant locations.

As seen in FIGS. 10A and 10B, with the clip housing 116 positioned at the puncture site P, the proximal pins 154, mounted in the caps 152, are positioned at the extreme right of the proximal driver slots 162 and of the circumferential portions of the proximal holder slots 164. The distal pins 150 are located at the distal end of the distal driver slots 166 and of the longitudinal portions of the distal holder slots 168.

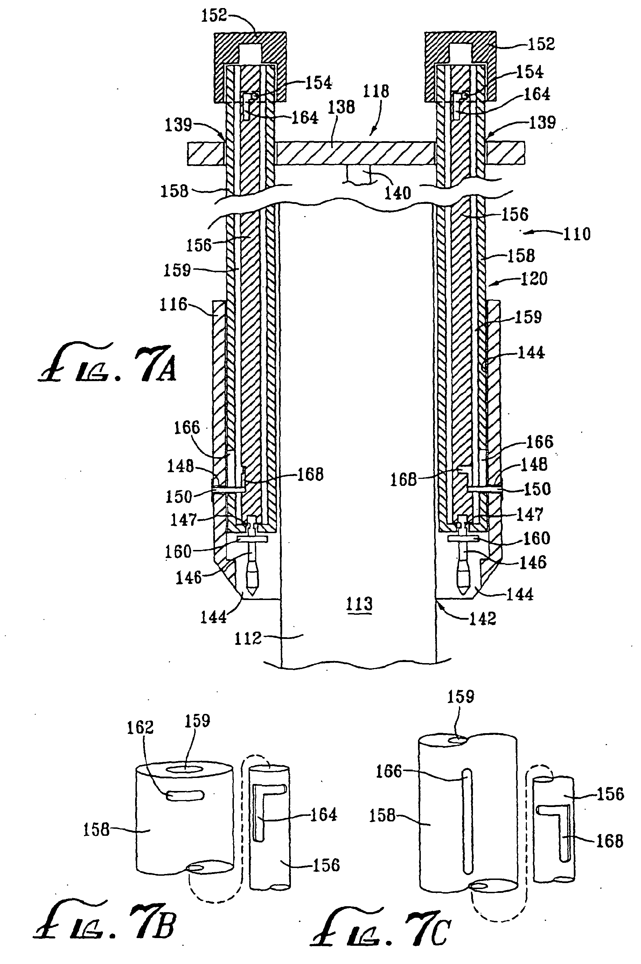

In FIGS. 11A and 11B, with the clip housing 116 held immobile, force is applied to the caps 152 to distally advance the clips 146 with respect to the clip housing 116. Specifically, the proximal pins 154 abut and apply force against the proximal slots 162, 164, thereby advancing the drivers 158 and the clip holders 156, as well as the attached clips 146 and the locking collars 180. The distal pins 150 move freely within the distal slots 166 and the longitudinal portions of the distal slots 168. Distal advancement of the clips 146 continues until the pins 150 abut against the proximal end of the longitudinal portions of the distal holder slots 168 of the clip holders 156. The drivers 158 likewise are restrained by their connection to the clip holders 156 via the proximal pins 154. The tissue-engaging members, i.e., spikes 174 and engagement elements 176, of the clips 146 contact and pierce the wall of the vessel V on opposite sides of the puncture site P.

As seen in FIGS. 12A and 12B, once the spikes have pierced the vessel wall, the locking collar drivers 158 are advanced distally while the clip housing 116 and the clip holders 156 remain stationary, thereby distally advancing the locking collars 180 down the exteriors of the clips 146 to draw the legs 170 and spikes 174 together to close the puncture P. Engagement elements 176 serve to retain the clips 146 within the vessel-wall during healing.

To achieve this advancement of the drivers 158 with respect to the clip holders 156, the caps 152 are rotated clockwise, as viewed from above, until the proximal pins 154 abut against the extreme left of the proximal slots 162, 164, thereby aligning the pins 154 with the longitudinal portions of the proximal holder slots 164. Force is then once again applied to the caps 152 to advance the drivers 158 and deform the clips 146 to their deployed configurations. Specifically, the proximal pins 154 abut and apply force to the proximal driver slots 162, thereby distally advancing the drivers 158. The pins 154 move freely within the longitudinal portions of the proximal holder slots 164 until they abut against the distal ends of the slots 164. Likewise, the distal driver slots 166 move freely until the distal pins 150 abut the proximal ends of the slots 166. In FIG. 12A, when the proximal pins 154 abut the slots 164 and the distal pins 150 abut the slots 166, the locking collars 180 have been driven down the exteriors of the clips 146, thereby deforming the clips 146 to draw the legs 170 together and close the puncture site.

In FIGS. 13A and 13B, with the clips 146 deformed to seal the puncture P, the clip holders 156 are detached from the clips 146 by snapping the clips 146 free at the narrowed regions 178.

At this point, or prior to detachment, a suitable biocompatible bioglue or tissue sealant optionally may be injected into the puncture tract, as discussed above, through the device port 132 or the side port 122, to aid in sealing the vascular puncture P. Alternatively, the bioglue or tissue sealant may be delivered through the backbleed path described above. The vascular device 110 then is withdrawn from the vessel wall, completing the procedure.

The clips 146 are detached from the clip holders 156 by rotating the caps 152 counterclockwise, as viewed from above. The proximal pins 154 of the caps 152 move freely within the proximal driver slots 162, but abut against the distal end of the longitudinal portions of the proximal holder slots 164 and cause the clip holders 156 to rotate with respect to the collar drivers 158. The distal pins 150 of the clip housing 116 move freely within the circumferential portions of the distal holder slots 168 during rotation of the clip holders 156. Meanwhile, the drivers 158 are restrained from rotation by the distal pins 150, which abut against the distal driver slots 166. The clips 146 do not rotate because the square cross section of the square clip bores 147 of the drivers 158 matches the substantially square cross section of the clips 146. Thus, since the drivers 158 are restrained from rotation, the clips 146 are as well. Non-square cross sections for the clips 146 and the bores 147 capable of performing the restraining function will be apparent to those of skill in the art and fall within the scope of the present invention.

Since the clips 146 are restrained while the clip holders 156 rotate, and since the proximal ends 172 of the clips 146 are attached to the clip holders 156, counterclockwise rotation of the caps 152 causes the clips 146 to snap at their weakest points, the narrowed regions 178. The vascular device 110 may then be removed from the patient to complete the procedure.

Although preferred illustrative embodiments of the present invention are described above, it will be evident to one skilled in the art that various changes and modifications may be made without departing from the invention. For example, with minor modifications, the vascular device 110 may be configured to carry the closure component 190 of FIGS. 9A and 9B, or any of a variety of alternative bioabsorbable and/or deformable clips. The proximal pins 154 may be formed integrally with the caps 152, and the distal pins 150 may be formed integrally with the clip housing 116. Any number of clips 146 may be used to close the vascular puncture.

Turning to FIG. 14, another example of an apparatus 210 is shown. The apparatus 210 includes 1 sheath 212 coupled to a hub 214, a closure component 216, and 1 closure actuator 218. The sheath 212, which may be an introducer sheath, a trocar, or a catheter, includes a central lumen 213 through which other devices (not shown) may be introduced into the vasculature, for example, to perform a diagnostic or interventional procedure such as angiography, angioplasty, or stenting, or to seal a puncture site.

The hub 214 is mounted on the proximal end of the sheath 212 and includes a side port 220, arc-shaped lumens 222, and a device port 224. The device port 224 communicates with the central lumen 213 of the sheath 212, and has a self-sealing elastomeric membrane 225 disposed across it. The self-sealing membrane 225, which may be formed from latex or a biocompatible synthetic rubber, may permit interventional devices to be introduced through the device port 224, while preventing blood loss through the central lumen 213. The side port 220 of hub 214 is also in communication with the central lumen 213, and is connected to a homeostatic port 226 via biocompatible tubing 28.

The closure component 216 includes a lumen 230 that receives the sheath 212. The closure component 216 is slidably disposed on the exterior of the sheath 12 and is movable from a stowed position, adjacent the hub 214, to a distal deployment position, where tines 217 of the closure component 216 are urged into engagement with tissue surrounding a vascular puncture. The closure component 216 includes at least two sharpened tips or tines 217. The tines 217 preferably include backbleed ports 232. The closure component 216 is rotatable within the arc-lumens 222 about the longitudinal axis of the sheath 212, so that, with tines 217 engaging tissue surrounding the vascular puncture, the closure component 216 may close the puncture.

The closure actuator 218 includes plunger 234 and tubes 236, which are configured to slidably pass through the arc lumens 222 of the hub 214. The proximal ends of the tubes 236 are coupled to backbleed bores 238 of the plunger 234. The distal ends of the tubes 236 are mounted, either permanently or detachably, in the closure component 216, so that movement of the plunger 234 causes corresponding proximal or distal movement of the closure component 216. Likewise, rotation of the plunger 234 causes corresponding rotation of the tubes 236 within the arc lumens 222, which, in turn, rotates the closure component 216 about the longitudinal axis of the sheath 212.

The plunger 234 also includes a device bore 240, coaxially aligned with the device port 224, and through which interventional devices or puncture sealants may be passed. As described in detail below, when the plunger 234 is moved to its proximal-most position, the closure component 216 is disposed adjacent to the hub 214 and preferably provides adequate clearance for interventional devices to be inserted through the device port 224 and the central lumen 213 into the patient's vasculature. When moved to its distal-most position, the plunger 234 causes the tubes 236 to urge the closure component 216 distally. Interventional devices or sealants then may be introduced through the device bore 240, the device port 224, and the central lumen 213 into the vasculature.

The backbleed bores 238 of the plunger 234 are in communication with backbleed lumens (not shown) within the tubes 236. The backbleed lumens of the tubes 236 are in communication with the backbleed ports 232 of the tines 217, thereby establishing a complete backbleed path through the ports 232, the lumens (not shown) of the tubes 236, and the bores 238. When the tines 217 of the closure component 216 pierce a vessel wall surrounding a vascular puncture, blood enters the backbleed ports 232 and exits through the backbleed bores 238, providing visual confirmation to a surgeon that the tines 217 are positioned within the vessel wall. The backbleed path thus enables the surgeon to determine when the closure component 216 has been sufficiently advanced to permit rotation of the closure component 216 to close the puncture, while reducing the risk that the closure component 216 is either short of the puncture site or is extended into the vessel.

In conjunction with closure of the puncture site caused by rotation of the closure component 216, a puncture sealant may be introduced to the puncture site to seal the site closed. The sealant may, for example, include an adhesive, such as a bioglue, tissue sealant, or clotting agent, delivered through the hemostatic port 226, the biocompatible tubing 228, the side port 220 and the central lumen 213 of the introducer sheath 212 to the vascular puncture to further help seal the vessel after puncture closure with the closure component 216. Alternatively, the adhesive may be delivered through the device port 224 or through the backbleed path described above. Instead of adhesives, the closure component 216 may further include the sealant, wherein the closure component 216 is left in place within the vessel until hemostasis naturally occurs. In addition, sutures (not shown) may be delivered through the central lumen 213 and/or thermal energy may be applied, for example, from electrical induction, infrared light, ultrasonic vibration, microwave or laser irradiation, and other methods.

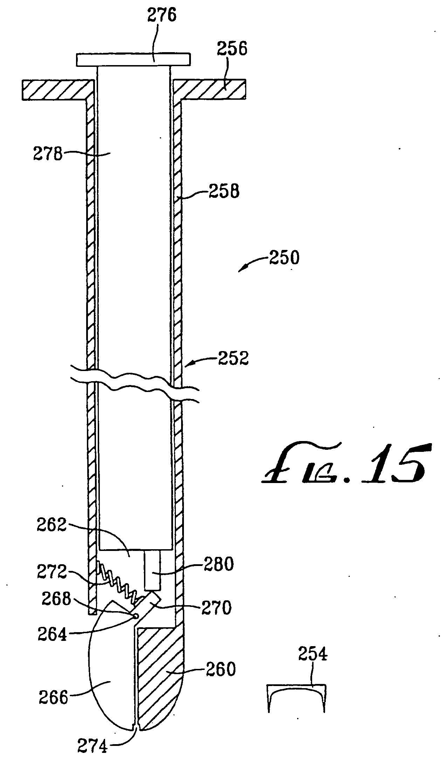

Turning to FIG. 15, an example of an alternative puncture sealing device 250 is shown. The sealing device 250 includes a delivery device 252 and a clip 254. The delivery device 252 includes a proximal end 256 attached to a tube 258. The tube 258 terminates at a first jaw 260 at its distal end and includes a lumen 262 and a pin 264. The pin 264 extends into the lumen 262 from an interior surface of the tube 258 and is disposed perpendicular to the longitudinal axis of the tube 258. The delivery device 252 also includes a second jaw 266 having a female connector 268 coupled to a pin 264, so that the second jaw 266 pivots about the pin 264. The second jaw 66 includes a moment arm 270, and a tension spring 272 is coupled to the moment arm 270 and to the interior surface of the tube 258 in a manner that biases the second jaw 266 against the first jaw 260.

The first and second jaws 260, 266 preferably form a channel 274 when biased against one another that is configured to receive the clip 254. The biasing force applied by the tension spring 272 holds the clip 254 within the channel 274, so that the clip 254 may be advanced into tissue surrounding a vascular puncture that has had its edges approximated by the closure component 216.

The delivery device 252 also includes a plunger 276 coupled to a pushrod 278 having a release arm 280. The pushrod 278 is received within the lumen 262 of the tube 258 such that the release arm 280 engages the moment arm 270.

Distal advancement of the pushrod 278, via application of force to the plunger 276, causes the release arm 280 to urge the moment arm 270 distally. This motion overcomes the biasing force applied by the tension spring 272 and causes the second jaw 266 to pivot about the pin 264. The second jaw 266 thus no longer contacts the first jaw 260, and the clip 254 is released from the channel 274. The tube 258, the first jaw 260, the second jaw 266, and the clip 254 of the sealing device 250 are preferably sized for introduction into a patient's vasculature through the device bore 240, the device port 224, and the lumen 213 of vascular device 210.

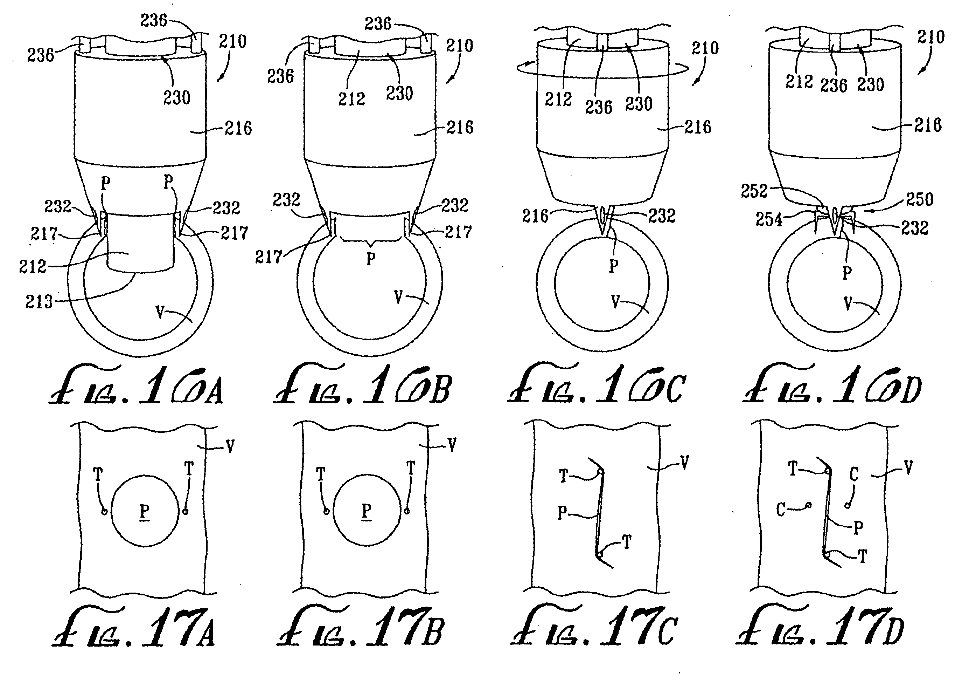

Referring to FIGS. 16A-16D through 17A-17D, in conjunction with FIGS. 14 and 15, a method of using the vascular device 210 with sealing device 250 is described. The sheath 212 is advanced through skin, fat, and muscle tissue into vessel V, through the vessel wall tissue surrounding vascular puncture P. With the plunger 234 and the tubes 236 of the actuator 218 in the proximal-most, fully retracted position, an interventional procedure may be performed by introducing one or more interventional devices, e.g. angioplasty balloons, stent delivery systems, atherectomy devices, etc., through the device port 224 and lumen 213 of the sheath 212, in accordance with well-known techniques. The side port 220 may be used to infuse fluids, e.g., contrast agents or medications, into the vessel through the sheath 212 during the interventional procedure.

Upon completion of the procedure, the vascular device 210 may be used to close the vascular puncture P. At this point, the closure actuator 218 and the closure component 216 are disposed in the proximal-most position, with the closure component 216 adjacent to the hub 214. The closure actuator 218 is advanced by urging the plunger 234 in the distal direction, thereby causing the tubes 236 to slide through the arc-shaped lumens 222 of the hub 214 and advance the closure component 216.

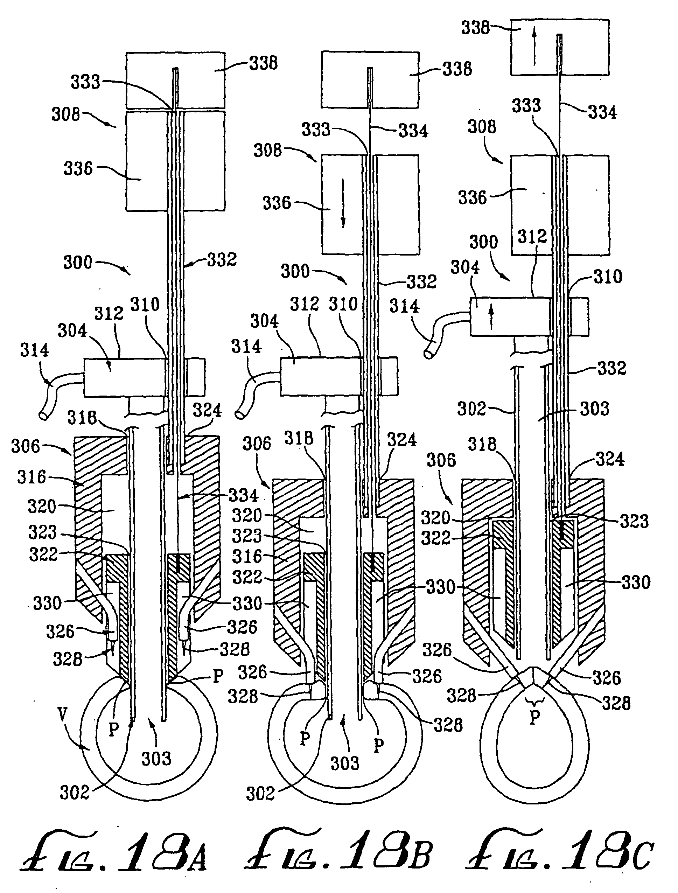

As seen in FIG. 16A, continued distal advancement of the plunger 234 causes the tines 217 at the distal end of the closure component 216 to pierce the tissue surrounding puncture P such that the backbleed ports 232 of the tines 217 directly communicate with the puncture wound. Tine punctures T in FIG. 17A represent the points at which the tines 217 enter vessel V. The presence of pressure in the vessel higher than atmospheric pressure causes blood to pass through the backbleed ports 232, through the backbleed lumens (not shown) of the tubes 236, and exit through the proximal ends of the backbleed bores 238, thus confirming that the tines 217 have engaged tissue around the puncture site and should not be advanced further.

In FIG. 16B, the sheath 12 is removed from the puncture P to facilitate closure of the puncture P. The closure actuator 218 is held stationary while the hub 214 is withdrawn proximally, thereby withdrawing the sheath 212 proximally from the puncture P. The puncture P remains open, as seen in FIG. 17B. With the sheath 212 no longer within the puncture P, the closure actuator 218 is rotated within the arc-shaped lumens 222 to rotate the closure component 216. Rotation of the closure component 216 causes the tines 217 to rotate and urge the puncture P closed, as seen in FIGS. 16C and 17C.

Upon closure of the puncture P, a sealant is introduced to seal the wound closed. The sealant may, for example, include an adhesive, such as a bioglue, tissue sealant, or clotting agent. In addition or alternatively, a suture may be used and/or thermal energy may be applied. The closure component 216 may remain in place within the vessel V until hemostasis naturally occurs. Alternatively, a sealing device, such as one of the clips described herein, may be applied.

FIGS. 16D and 17D show the apparatus 210 used in conjunction with the sealing device 250 of FIG. 15. With the clip 254 disposed in the channel 274 of the delivery device 252, the delivery device 252 may be delivered to the vessel V through the device bore 240 of the closure actuator 218, the device port 224 of the hub 214, and the central lumen 213 of the sheath 212. The clip 254 punctures the vessel V at tissue surrounding the closed puncture P, creating clip punctures C and sealing the, puncture P. The pushrod 278 of the delivery device 252 is then actuated to separate the second jaw 266 from the first jaw 260 to release the clip 254 from the delivery device 252. The apparatus 210 and the delivery device 252 are then removed from the patient to complete the procedure. The clip 254 maintains closure until hemostasis occurs and is preferably bioabsorbable so that no foreign materials are permanently implanted in the patient's body. Additional clips may also be implanted, if desired or required.

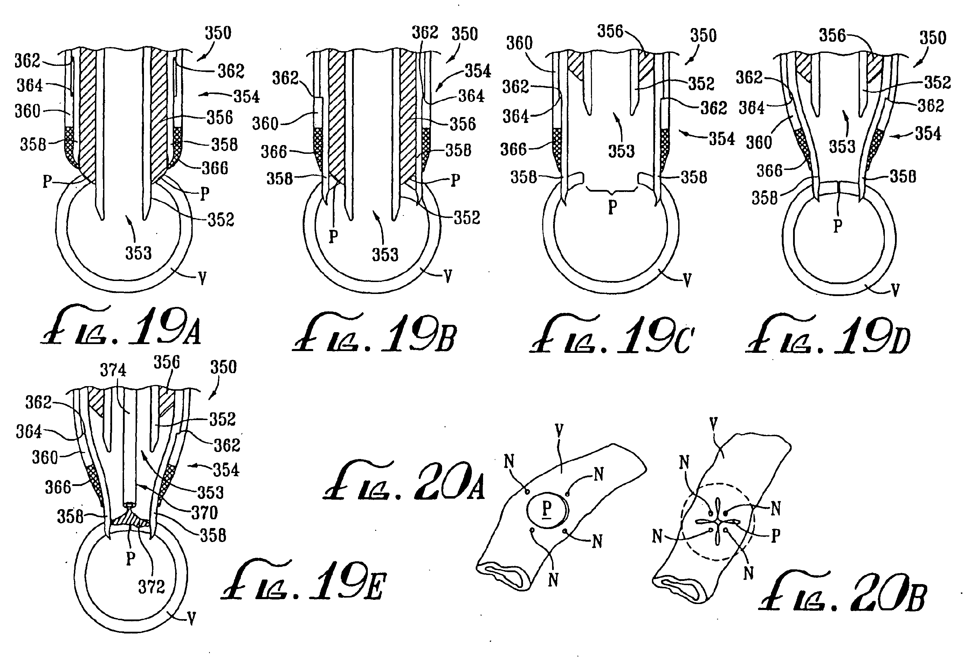

With reference now to FIGS. 18A-18C, an example of an integrated vascular apparatus 300 is described. The Apparatus 300 includes a sheath 302 coupled to a hub 304, a closure component 306, and a closure actuator 308. Similar to sheath 112 described above, the sheath 302 may be an introducer sheath, a trocar, or a catheter, and includes a central lumen 303 through which other devices (not shown) may be introduced into the vasculature, for example, to perform a diagnostic or interventional procedure such as angiography, angioplasty, or stenting, or to seal a puncture site. The hub 304 includes a bore 310 that slidably receives the actuator 308, a device port 312, and a side port 314. The device port 312 is in communication with the central lumen 303 of the sheath 302 and permits introduction of interventional devices while preventing blood loss through the central lumen 303.

The closure component 306 includes an outer housing 316 having a lumen 318 configured to slidably receive the sheath 302, a bore 320 for slidably receiving an inner housing 322, a lumen 324 adapted to receive the closure actuator 308, and needles or prongs 326 with sharpened tips 328. The inner housing 322 has a lumen 323 for receiving the sheath 302 and channels 330 for receiving the prongs 326. The closure component 306 includes at least two prongs 326, and preferably includes four.

The closure actuator 308 includes an actuation tube 332 having a lumen 333, an actuation rod 334 disposed within the actuation tube 332, a first plunger 336 coupled to the proximal end of the tube 332, and a second plunger 338 coupled to the proximal end of the rod 334. The distal end of the tube 332 is affixed, either permanently or detachably, in the lumen 324 to the outer housing 316 of the closure component 306, while the distal end of the rod 334 is coupled to the inner housing 322.

To perform an interventional procedure through the central lumen 303 of the sheath 302, the sheath 302 is advanced through skin, fat, and muscle tissue into vessel V, through vascular puncture P, in accordance with well-known techniques. With the closure component 306 in its proximal-most, fully retracted position adjacent the hub 304, the interventional procedure is then performed by introducing one or more interventional devices, e.g. angioplasty balloons, stent delivery systems, atherectomy devices, etc., through the device port 312 and the lumen 303 of the sheath 302, again in accordance with well-known techniques. The side port 314 may be used to infuse fluids, e.g., contrast agents or medications, into the vessel V through the sheath 302 during the interventional procedure.

Upon completion of the procedure, the apparatus 300 may be used to close the vessel V. The closure component 306 is advanced distally by urging the plungers 336, 338 distally. The inner housing 322 is only partially received within the bore 320 of the outer housing 316 such that the prongs 326 are elastically deformed and received within the channels 330. As shown in FIG. 18A, the closure component 306 is advanced until the inner housing 322 abuts against the vessel V, as may be determined, for example, with a backbleed indicator (not shown).

In FIG. 18B, the first plunger 336 is urged distally to distally advance the actuation tube 332 and the outer housing 316, while the second plunger 338 and the sheath 302 are held stationary. Advancement of the outer housing 316 advances the sharpened tips 328 of the prongs 326 into tissue surrounding puncture P.

In FIG. 18C, the sheath 302 and the second plunger 338 are retracted proximally to draw the sheath 302 out of the vessel V and to draw the inner housing 322 completely within the bore 320 of the outer housing 316. Proximally retracting the inner housing 322 via the actuation rod 334 and the second plunger 338 removes the prongs 326 of the outer housing 316 from the channels 330 of the inner housing 322. The prongs 326 resiliently contract to a lower stress configuration, thereby drawing opposing sides of the puncture P together and closing the wound. A sealant, for example, the clip 254 of FIG. 15, may then be introduced to the closed puncture to seal the site closed, as described above. Alternatively, RF current, supplied by an RF generator (not shown), may be applied across the opposed tips 328, which may act as bipolar electrodes.

Referring to FIGS. 19A-19E, as well as FIGS. 20A and 20B, a still further example of an apparatus 350 is shown. FIGS. 19A-19E depict a closure component 354 of the integrated vascular device in use at vascular puncture P within vessel V. The apparatus 350 includes a sheath 352 coupled to a hub (not shown), a closure component 354, and a closure actuator (not shown). Various closure actuators for use with the closure component 354 will be apparent to those of skill in the art from the foregoing embodiments.

The sheath 352 may be an introducer sheath, a trocar, or a catheter, and may include a central lumen 353 through which other devices (not shown) may be introduced into the vasculature, for example, to perform a diagnostic or interventional procedure, such as angiography, angioplasty, or stenting, or to seal a puncture site, similar to the previous embodiments. The closure component 354 includes a spacer 356, needles 358, and a needle cover 360. The spacer 356 is coaxially and slidably disposed about the exterior of the sheath 352, and preferably has an annular diameter of about one millimeter (1mm) to ensure that the needles 358 engage the tissue surrounding the puncture P rather than enter the puncture, so that the needles 358 are able to draw the wound closed, as described further below. The needles 358 are disposed between the spacer 356 and the cover 360 during advancement to the puncture P. The needles 358 include ledges 362, which act as positive stops to prevent excessive advancement of the needles 358 with respect to the cover 360, which includes a corresponding annular ledge 364. The cover 360 also includes an elastic segment 366 configured to elastically deform the needles 358. The closure component 354 includes at least two needles 158, and preferably includes four. The needles 358 may further include engagement elements (not shown), such as barbs or hooks, to assist in gripping tissue.

As shown in FIG. 19A, the sheath 352 may be advanced through skin, fat, and muscle tissue into the vessel V, through vascular puncture P, in accordance with well-known techniques. With the closure component 354 in its proximal-most, fully retracted position adjacent the hub 304, an interventional procedure is performed through the central lumen 353 of the sheath 352 by introducing one or more interventional devices through the lumen into the patient's vasculature. The closure component 354 is then advanced via the closure actuator 308 until it abuts against the vessel V, as may be determined, for example, with a backbleed indicator, such as that described previously. The cover 360 protects the needles 358 and prevents snagging of tissue as the closure component 354 is distally advanced down the sheath 352 and through skin, fat, and muscle tissue. The spacer 356 retains the needles 358 in a position away from the edge of the puncture P.

In FIG. 19B, the needles 358 are distally advanced with respect to the needle cover 360 until the ledge 362 abuts the ledge 364. The needles 358 deflect the elastic segment 366 of the cover 360 outward and pierce the tissue surrounding the puncture P. FIG. 20A depicts, in isometric view, the segment of the vessel V surrounding the puncture P. With a needle arrangement including four needles 358, the needles 358 create needle punctures N surrounding the vascular puncture P. The sheath 352 and spacer 356 are then retracted proximally and removed from the vessel V, as shown in FIG. 19C. As depicted in FIGS. 19D and 20B, the elastic segment 366 of the needle cover 360 resiliently contracts, thereby drawing the needles 358 together and approximating the edges of the wound.

A sealant, such as a bioglue, tissue sealant, or clotting agent, may then be introduced to the puncture site to seal the wound closed. Alternatively, the closure component 354 may be maintained in position until hemostasis occurs naturally, or sutures (not shown) may be introduced through the central lumen 353. In addition, or in the alternative, RF energy may be applied across the needles 358, as described above, or a clip, such as clip 254 of the sealing device 250 of FIG. 15, may be applied. Thermal energy from electrical induction, infrared light, ultrasonic vibration, microwave or laser irradiation, and other means may also be used to seal the puncture. Illustratively, FIG. 19E depicts a sealing device 370, including adhesive 372, being delivered through the central lumen 353 within the sheath 374. After sufficient time for adhesive 372 to set, the apparatus 350 may be removed from the vessel V.

Although preferred illustrative embodiments of the present invention are described above, it will be evident to one skilled in the art that various changes and modifications may be made without departing from the invention. For example, with minor modifications, vascular device 10 may be configured to carry the clip 90 of FIGS. 4, or any of a variety of alternative expandable resilient clips. It is intended in the appended claims to cover all such changes and modifications that fall within the scope of the invention.

## Claims

1. An apparatus (10) for closing an opening in tissue, comprising:
a tubular member (12) having proximal and distal regions and an exterior surface;
a housing (16) slidably disposed on the exterior surface of the tubular member (12), the housing (16) including a portion defining an annular-shaped chamber;
at least one deformable clip (146) releasably disposed within the chamber, the housing preventing the clip (146) from snagging on tissue during advancement of the housing (16), the deformable clip (146) having first and second curved legs (170) each leg (170) having a common first end (172) and a separate second end (174), the second ends (174) spaced apart from each other; **characterized in that**
a locking collar (180) is slidably disposed on the clip (146) adjacent the first end (172), wherein the locking collar (180) is advanceable along the clip (146) to deform the clip (146) to a deployed configuration.

2. The apparatus according to claim 1, further including a second clip (146), the second clip including a second locking collar (180).

3. The apparatus according to claim 2, wherein the second clip (146) is spaced radially apart from the first clip (146).

4. The apparatus according to claims 1 and 2, wherein the clip (146) is constructed of a bioabsorbable material.

5. The apparatus according to claim 4, wherein the locking collar (180) is constructed of a bioabsorbable material.

6. The apparatus according to claims 1 and 2, wherein the clip (146) further includes a sharpened distal end (174) configured to puncture tissue.

7. The apparatus according to claim 2, wherein the second clip (146) is spaced radially apart from the first clip (146).

8. The apparatus according to claims 1 and 2, further including at least one locking collar driver (158) associated with the locking collar (180).

9. The apparatus according to claims 1 and 2, wherein the first end (172) of the clip (146) is frangible.

## Patentansprüche

1. Vorrichtung (10) zum Verschließen einer Öffnung in einem Gewebe, die aufweist:
ein röhrenförmiges Element (12) mit einem proximalen und einem distalen Bereich und einer Außenfläche,
ein Gehäuse (16), das verschiebbar auf der Außenfläche des röhrenförmigen Elementes (12) angeordnet ist, wobei das Gehäuse (16) einen Abschnitt aufweist, der eine ringförmige Kammer definiert,
wenigstens einen verformbaren Clip (146), der lösbar innerhalb der Kammer angeordnet ist, wobei das Gehäuse verhindert, dass der Clip (146) während Vorrückens des Gehäuses (16) am Gewebe hängen bleibt, wobei der verformbare Clip (146) ein erstes und ein zweites gebogenes Bein (170) aufweist, wobei jedes Bein (170) ein gemeinsames erstes Ende (172) und ein separates zweites Ende (174) aufweist, wobei die zweiten Enden (174) voneinander beabstandet sind,
**dadurch gekennzeichnet, dass** eine Verriegelungsmanschette (180) neben dem ersten Ende (172) verschiebbar auf dem Clip (146) angeordnet ist, wobei die Verriegelungsmanschette (180) entlang des Clips (146) verrückbar ist, um den Clip (146) in eine Einsetz-Konfiguration zu verformen.

2. Vorrichtung nach Anspruch 1, die ferner einen zweiten Clip (146) aufweist, wobei der zweite Clip eine zweite Verriegelungsmanschette (180) aufweist.

3. Vorrichtung nach Anspruch 2, wobei der zweite Clip (146) von dem ersten Clip (146) radial beabstandet ist.

4. Vorrichtung nach den Ansprüchen 1 und 2, wobei der Clip (146) aus einem bioabsorbierbaren Material ausgebildet ist.

5. Vorrichtung nach Anspruch 4, wobei die Verriegelungsmanschette (180) aus einem bioabsorbierbaren Material ausgebildet ist.

6. Vorrichtung nach den Ansprüchen 1 und 2, wobei der Clip (146) ferner ein spitzes distales Ende (174) aufweist, das zur Gewebepunktion konfiguriert ist.

7. Vorrichtung nach Anspruch 2, wobei der zweite Clip (146) von dem ersten Clip (146) radial beabstandet ist.

8. Vorrichtung nach den Ansprüchen 1 und 2, die ferner wenigstens einen Verriegelungsmanschetten-Treiber (158) aufweist, der mit der Verriegelungsmanschette (180) verbunden ist.

9. Vorrichtung nach den Ansprüchen 1 und 2, wobei das erste Ende (172) des Clips (146) zerbrechlich ist.

## Revendications

1. Appareil (10) de fermeture d'une ouverture dans un tissu, comprenant :
un élément tubulaire (12) ayant des régions proximale et distale et une surface extérieure ;
un boîtier (16) disposé, pour coulisser, sur la surface extérieure de l'élément tubulaire (12), le boîtier (16) comportant une portion définissant une chambre de forme annulaire ;
au moins une agrafe déformable (146) disposée, de manière amovible, dans la chambre, le boîtier empêchant que l'agrafe (146) n'accroche un tissu au cours de la progression du boîtier (16), l'agrafe déformable (146) ayant des première et deuxième pattes incurvées (170), chaque patte (170) ayant une première extrémité commune (172) et une deuxième extrémité séparée (174), les deuxièmes extrémités (174) étant espacées les unes des autres ; **caractérisée en ce que** :
une bague de blocage (180) est disposée, pour coulisser, sur l'agrafe (146), de façon adjacente à la première extrémité (172), grâce à quoi la bague de blocage (180) peut progresser le long de l'agrafe (146) pour déformer l'agrafe (146) afin de lui donner une configuration déployée.

2. Appareil selon la revendication 1, incluant en outre une deuxième agrafe (146), la deuxième agrafe incluant une deuxième bague de blocage (180).

3. Appareil selon la revendication 2, dans lequel la deuxième agrafe (146) est espacée radialement de la première agrafe (146).

4. Appareil selon les revendications 1 et 2, dans lequel l'agrafe (146) est élaborée à partir d'un matériau bioabsorbable.

5. Appareil selon la revendication 4, dans lequel la bague de blocage (180) est élaborée à partir d'un matériau bioabsorbable.

6. Appareil selon les revendications 1 et 2, dans lequel l'agrafe (146) inclut en outre une extrémité distale effilée (174), configurée pour ponctionner le tissu.

7. Appareil selon la revendication 2, dans lequel l'agrafe (146) est espacée radialement de la première agrafe (146).

8. Appareil selon les revendications 1 et 2, incluant en outre au moins un dispositif d'entraînement de la bague de blocage (158) associé à la bague de blocage (180).

9. Appareil selon les revendications 1 et 2, dans lequel la première extrémité (172) de l'agrafe (146) est sécable.
